# EUROPÄISCHE PATENTANMELDUNG

(11) **EP 2 883 554 A1**
(43) Veröffentlichungstag der Anmeldung: **17.06.2015**
(21) Anmeldenummer: 13197577.3
(22) Anmeldetag: 16.12.2013
(51) Int. Cl.: A61L 31/04, A61L 31/06, A61L 31/16, C08L 67/04, C08L 75/04, D01D 5/00, D01D 5/08

(54) **Herstellung von resorbierbaren Polymerrohren aus Fäden**

(71) Anmelder: MeKo Laserstrahl-Materialbearbeitungen e.K., 31157 Sarstedt (DE)
(72) Erfinder: Dohse, Jakob, 30171 Hannover (DE); Hinrichs, Bernd, 31319 Sehnde (DE)
(74) Vertreter: Arth, Hans-Lothar

(57) **Zusammenfassung**

Die vorliegende Erfindung betrifft Polymerrohre, bevorzugt aus bioresorbierbaren Polymeren, hergestellt aus mindestens einem Polymerfaden, wobei der Polymerfaden einen Durchmesser von ≤ 50 µm aufweist, sowie Verfahren zur Herstellung dieser Polymerrohre, welche insbesondere für die Herstellung von Stents geeignet sind.

## Beschreibung

Die vorliegende Erfindung betrifft Polymerrohre, bevorzugt aus bioresorbierbaren Polymeren, hergestellt aus mindestens einem Polymerfaden, wobei der Polymerfaden einen Durchmesser von ≤ 50 µm aufweist, sowie ein Verfahren zur Herstellung dieser Polymerrohre, welche insbesondere für die Herstellung von Stents geeignet sind.

Die Implantation von Gefäßstützen wie beispielsweise Stents ist heutzutage ein gängiger chirurgischer Eingriff zur Behandlung von Stenosen. Sie werden üblicherweise aus Metalllegierungen, wie nichtrostendem Edelstahl, Kobalt-Chromlegierungen oder Nitinol hergestellt. Derartige Metallstents sind in großer Zahl bekannt und haben sich in der Praxis bewährt. Zum einen sollen derartige Metallstents aufgrund ihrer Metallstruktur und radialen Festigkeit gewährleisten, dass die Blutgefäße nach einer Aufdehnung und Stent-Implantation offen bleiben und der Blutfluss durch die Gefäße dauerhaft sichergestellt ist. Zum anderen dienen Stents in der Krebsbehandlung dazu, durch bösartige Tumoren verursachte Verengungen von Atemwegen (Luftröhre), Gallenwegen oder der Speiseröhre zu verhindern oder nach einer Aufdehnung offenzuhalten.

Stents werden derzeit in zwei Grundtypen eingeteilt: dauerhafte (biostabile) und resorbierbare (degradierbare) Stents. Dauerhafte Stents sind so ausgestaltet, dass sie im Gefäß für einen unbestimmten Zeitraum verbleiben können. Resorbierbare Stents werden hingegen über einen vorbestimmten Zeitraum hinweg im Gefäß abgebaut.

Neuere Untersuchungen haben gezeigt, dass Gefäßstenosen nicht permanent durch eine Endoprothese, insbesondere in Form eines Stents, gestützt werden müssen. Es ist vollkommen ausreichend, das Blutgefäß für einen begrenzten Zeitraum zu stützen, bis das traumatisierte Gewebe des Gefäßes verheilt und sich die glatten Gefäßmuskelzellen regeneriert haben. Sie übernehmen dann wieder die Aufgabe, das Blutgefäß offen zu halten, weshalb der Stent nicht länger erforderlich ist und im Gefäßlumen verbleiben muss. Diese zeitlich begrenzte Funktion der Gefäßstützung wird durch sogenannte resorbierbare Stents erfüllt, die im Körper abgebaut werden.

Resorbierbare Stents haben den Vorteil, dass das körperfremde Material nicht dauerhaft im Gefäß verbleibt und die Gefahr einer negativen Gefäßreaktion wie einer Restenose somit zeitlich begrenzt wird. Bei Kindern besteht für resorbierbare Stents zusätzlich der Vorteil, dass die Gefäße in ihrem Wachstum nicht durch eine permanente Gefäßstütze gehemmt werden.

Derzeit sind zwei grundsätzlich unterschiedliche Materialien für die bioresorbierbaren Stents im Einsatz bzw. in der klinischen Untersuchung:
- Biopolymere, d.h. resorbierbare Polymere, insbesondere Polylactide
- Metalle, insbesondere Magnesiumlegierungen

Bezüglich der bioresorbierbaren Metall-Stents hat bislang nur die Firma Biotronik klinische Studien mit einer bioresorbierbaren Magnesium-Legierung durchgeführt. Die Firma Lifetech (China) hat hingegen erste Tierversuche mit einem eisenbasierten, resorbierbaren Stent durchgeführt. Von anderen Firmen sind keine klinischen Studien oder präklinischen Studien am Tiermodel bekannt. Obgleich grundsätzlich das angestrebte Ziel einer Resorption des implantierten Stents erreicht wird, besteht bei Magnesiumlegierungen bislang das Problem eines zeitlich nicht definierten Abbaus des Stents. Je nach Legierung ist der Materialabbau starken Schwankungen unterworfen, schwer kontrollierbar und im Allgemeinen zu schnell, um ein sicheres Einwachsen des Stents in die Gefäßwände und Übernahme der Stützfunktion bis zur Regeneration des Gefäßabschnitts zu gewährleisten. Durch eine unkontrollierte Degradation können sich Bruchstücke des Stents ablösen und Thrombosen verursachen.

Die meisten Entwicklungen resorbierbarer Stents konzentrieren sich aber auf Biopolymere und hierbei auf Polylactide, auch Polymilchsäuren genannt (kurz PLA, vom englischen Wort *polylactic acid).* Sie sind technische Biopolymere, gehören zu den Polyestern und sind aus vielen, chemisch aneinander gebundenen Milchsäuremolekülen aufgebaut.

Von den Polylactiden werden insbesondere das Poly-L-lactid (PLLA) und das Polylactid-co-Glycolid (PLGA) für resorbierbare Stents verwendet.

Als Erste hat Kyoto Medical im Nov. 2007 eine CE-Zulassung für einen unbeschichteten PLLA Stent Igaki-Tamai^{®} erhalten. Später gelang Abbott für den Absorb^{®} die CE-Zulassung; ein PLLA Stent mit Everolimus als Wirkstoff in einer Poly-D,L-Lactid (PDLLA) Beschichtung. Im Jahr 2013 hat Elixir die Zulassung für seinen PLLA basierten Stent Desolve^{®} mit einer Novolimus enthaltenden Beschichtung erhalten.

Polylactide sind problemlos verfügbar. Es gibt mehrere Hersteller mit langjährigen Erfahrungen. Das Material gilt als körperverträglich. Aus der Chirurgie ist bioresorbierbares Polylactid-Nahtmaterial seit Jahren bekannt und im Einsatz. Folglich existieren keine Einwände Polylactide als Implantatmaterial zu verwenden und es müssen keine umfangreichen Biokompatibilitäts-Untersuchungen durchgeführt werden. Die Resorbierbarkeit lässt sich durch Wahl der geeigneten Polylactide bzw. Materialmischungen (Co-polymere) genau einstellen. Derzeit strebt man eine völlige Degradation, d.h. einen völligen Abbau der Stents bzw. Stentstreben innerhalb von 18 Monaten (1,5 Jahre) nach Implantation an.

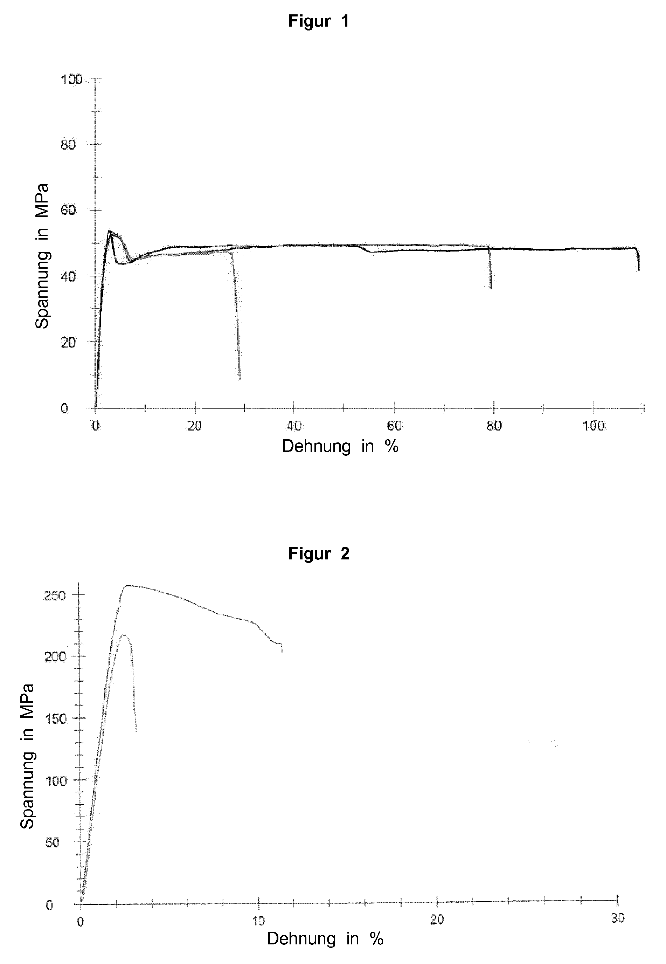

Die Polylactide PLLA und PLGA weisen als Werkstoff für Stents, das heißt in Form eines Rohrs, im Allgemeinen eine hohe Bruchdehnung auf. Bruchdehnungen bis zu 100 % für ein handelsübliches PLLA Rohr (ø 1,8 x 0,15 mm) sind problemlos erreichbar; allerdings mit dem Nachteil, dass die Festigkeit dieser Rohre nur 50 - 60 MPa beträgt (siehe Figur 1). Festigkeitssteigernde Maßnahmen führen hingegen zu einer Verringerung der Bruchdehnung. Den Vorteilen der Verwendung von Polylactiden steht somit auch eine Reihe von Nachteilen gegenüber.

Als größter Nachteil der Polylactide ist die begrenzte Festigkeit des Materials zu nennen, weshalb Verfahren zur Festigkeitssteigerung zum Einsatz kommen. PLLA Rohre zeigen bei Zugversuchen eine Festigkeit von unter 60 MPa (typischerweise 50 MPa). Aufgrund der geringen Festigkeit der Polylactide sind hohe Wandstärken bei den Stentrohlingen (Polymerrohre) respektive den Stentstreben notwendig, damit die Stents eine ausreichende radiale Festigkeit zur Gefäßstützung aufweisen.

Große Anstrengungen werden unternommen, um die Festigkeit der Polymere bzw. der Rohre zu steigern. Dennoch betragen heutzutage die Rohr- bzw. Stegwandstärken mindestens 150 µm, um eine ausreichende radiale Festigkeit zu gewährleisten. Mit diesen Wandstärken sind kleine Gefäßdurchmesser nicht erreichbar. Diese massiven Stege stehen im Widerspruch zu dem Trend, nicht resorbierbare Stents mit immer dünneren Stegen herzustellen mit Stegquerschnitten von teilweise nur noch 60 µm und darunter. Man erwartet von diesen filigranen Stents bessere Heilungschancen für das Gefäß.

Die festigkeitssteigernden Maßnahmen führen wiederum zu einer geringeren Bruchdehnung der Polymere, weshalb bereits geringe Überdehnungen der Polylactid-Stents zu Brüchen einzelner Stege führen. Trotz der hohen Stegquerschnitte ist die Festigkeit der Stentstreben oder auch Stege begrenzt, was in Kombination mit der geringen Bruchdehnung (der Bruchempfindlichkeit) zu einer kostenaufwändigen und langwierigen Prozedur bei Stentimplantationen führt.

So wird empfohlen, die PLLA Stents nicht direkt zu implantieren ("direct stenting"), sondern mit Hilfe einer Ballondilatation das stenotisierte Gefäß zuvor aufzudehnen ("pre-dilatation"). Teilweise wird noch eine Atherektomie durchgeführt, d.h. mittels minimal-invasiver Methoden werden die harten Ablagerungen in den Arterien entfernt. Danach soll der Gefäßdurchmesser vorzugsweise mittels der IVUS oder OCT Methode präzise vermessen werden, um die richtige Stentgröße auszuwählen und damit eine Über- oder Unterdehnung des Polylactid Stents zu vermeiden. Schließlich wird der Polylactid-Stent dann in das bereits geöffnete und vorbereitete Gefäß eingesetzt. Eine Post-Dilatation des implantierten Stents mit einem noncompliant Ballonkatheter wird zusätzlich empfohlen.

Als weiterer Nachteil der Polylactid Stents sei hier die begrenzte Lagerfähigkeit aufgrund der Empfindlichkeit des Materials gegenüber Temperatur und Feuchtigkeit genannt. Unerwünschte Zersetzungsprozesse können frühzeitig vor der Implantation beginnen.

Polymere neigen zudem allgemein zum Kriechen, insbesondere gedehnte Strukturen aus den Polymeren zeigen dieses Verhalten. Auf den Ballonkatheter gecrimpte Stents dehnen sich ganz langsam und allmählich wieder etwas auf, wodurch der Stent keinen festen Sitz mehr auf dem Ballonkatheter hat. Im Gefäß aufgedehnte Stents ziehen sich wieder leicht zusammen und verringern damit den Gefäßquerschnitt respektive den Blutfluss.

Rohre für die Herstellung von Gefäßimplantaten bzw. Stents weisen kleine Durchmesser von typischerweise unter 3,0 mm auf. Bestehen diese Rohre aus Biopolymeren, werden sie bevorzugt durch Extrusion hergestellt. Bei einer Herstellung durch Extrusion (Strangpressen) wird ein hochreines trockenes Polylactid-Granulat in kleinen Extrudern zu einer dickflüssigen Masse erschmolzen und durch einen Schneckentrieb kontinuierlich unter hohem Druck aus einer Düse als Rohr ausgepresst.

Ein weiteres Herstellungsverfahren ist das Spritzgießen (Spritzguss). Dazu wird mit einer Spritzgießmaschine der jeweilige Werkstoff in einer Spritzeinheit plastifiziert und in ein Spritzgießwerkzeug eingespritzt. Der Hohlraum, die Kavität, des Werkzeugs bestimmt die Form und die Oberflächenstruktur des fertigen Teils. Alternativ können Polymerrohre auch aus Hohlkörpern gezogen werden: Rohrziehprozess.

Den Herstellungsverfahren Extrusion und Spritzgießen ist gemein, dass die langkettigen Polylactid-Molekülketten weitgehend ungerichtet sind bzw. leicht in Pressrichtung (axiale Rohrrichtung) vororientiert sind und die Rohre in einem weitgehend spannungsfreien Zustand vorliegen. Die Zugfestigkeiten der Rohre sind mit 50 bis 60 MPa gering. Ausreichende radiale Festigkeiten der Stents lassen sich mit diesen Polylactid-Rohren nur durch hohe Wandstärken bzw. große Querschnitte der Stentstreben erreichen.

Daher werden festigkeitssteigernde Maßnahmen vorgenommen. Von thermoplastischen Kunststoffen ist bekannt, dass die Materialfestigkeiten durch Verstrecken gesteigert werden können. Dieses Verfahren wird standardmäßig vielfach in der industriellen Fertigung von Kunststoffprodukten angewandt.

Beim Verstrecken gleiten die langkettigen Polymermoleküle aufeinander, werden entknäuelt und in Dehnrichtung zueinander ausgerichtet. Sie liegen zunehmend parallel zueinander. Die Orientierung der Molekülketten ist gleichbedeutend mit einer partiellen Kristallisation (Teilkristallisation), wobei im Material gleichzeitig Spannungen induziert werden (innere Spannungen). Die teilkristalline Anordnung sollte durch Sekundärbindungen (Van der Waals Kräfte, Dipol-Dipol-Bindungen oder Wasserstoffbrücken) stabilisiert werden, um den Spannungsabbau, d.h. die Tendenz zum Kriechen in Richtung der Ursprungsform zu vermeiden. Das Verstrecken muss nicht zwingend bei Raumtemperatur erfolgen. So erfolgt das Dehnen der PLLA Rohre bei Temperaturen von typischerweise 50 bis 100°C.

Dies bedeutet, dass beim Verstrecken zwei Prozesse gleichzeitig ablaufen: die Moleküle werden in Dehnrichtung ausgerichtet und die entstehenden kristallinen Bereiche werden gleichgerichtet.

Die Polymerrohre können zum Verstrecken über einem Dorn geweitet werden, so dass eine Festigkeitssteigerung eintritt. Eine andere Methode kommt aus der Ballonkatheterherstellung. Mit den gleichen Anlagen und Vorrichtungen zum Aufblasen (Ausformen) eines Ballons für einen Ballonkatheter wird das PLLA-Rohr in einer Form auf einen größeren Durchmesser radial aufgedehnt. Ein ähnliches Verfahren wird in US2011/0062638 A1 offenbart. Figur 2 zeigt das Spannungs-Dehnungsdiagramm eines mit diesem Verfahren radial aufgedehnten, d.h. verfestigten PLLA Rohres aus dem Stand der Technik. Es ist eine deutlich höhere Festigkeit, aber auch die verringerte Bruchdehnung zu erkennen. Die geringere Bruchdehnung führt zu einer begrenzten Dehnbarkeit der Polylactid-Stents und zu einer Bruchgefahr bei nur geringer Überdehnung über den Nominaldurchmesser des Stents. Der Nominaldurchmesser eines Stents bezeichnet den Innendurchmesser des Stents im aufgedehnten (dilatierten), implantierten Zustand.

Wie dargelegt weisen Polylactide als Stentmaterial den entscheidenden Nachteil einer begrenzten Festigkeit auf, weshalb Verfahren zur Festigkeitssteigerung zum Einsatz kommen. Allerdings führen die derzeitigen, festigkeitssteigernden Methoden zu einer begrenzten Dehnbarkeit und damit starken Einschränkungen bei der Verwendung der Biopolymerstents.

Ziel der vorliegenden Anmeldung ist es daher, durch geeignete Maßnahmen und Verfahren die Festigkeit von Polymerrohren und daraus hergestellten Stents oder anderen Gefäßimplantaten zu steigern. Die erfindungsgemäße Herstellung von Polymerrohren beseitigt die starken Einschränkungen bzw. verbessert die Eigenschaften der Polymerrohre, die dadurch für die Herstellung von Stents besonders geeignet sind.

Aufgabe der vorliegenden Erfindung ist es ein Polymerrohr bereitzustellen, welches eine erhöhte radiale Festigkeit bei kaum verminderter Bruchdehnung aufweist und daher für die Herstellung von Stents besonders geeignet ist.

Diese Aufgabe wird erfindungsgemäß durch die technische Lehre der unabhängigen Ansprüche gelöst. Weitere vorteilhafte Ausgestaltungen der Erfindung ergeben sich aus den abhängigen Ansprüchen, der Beschreibung, den Figuren sowie den Beispielen.

Die vorliegende Erfindung ist gerichtet auf ein Polymerrohr hergestellt aus mindestens einem Polymerfaden, wobei der Polymerfaden einen Durchmesser von ≤ 50 µm aufweist. Der Durchmesser des erfindungsgemäß genutzten Polymerfadens ist ≤ 50 µm und bevorzugt ≤ 30 µm, weiter bevorzugt ≤ 20 µm, weiter bevorzugt ≤ 15 µm, weiter bevorzugt ≤ 12 µm, weiter bevorzugt ≤ 10 µm, weiter bevorzugt ≤ 8 µm, noch weiter bevorzugt ≤ 5 µm und besonders bevorzugt ≤ 2,5 µm. Der Durchmesser des Polymerfadens liegt bevorzugt in folgenden Bereichen: zwischen 50 nm und 50 µm, weiter bevorzugt zwischen 100 nm und 20 µm, weiter bevorzugt zwischen 150 nm und 10 µm, noch weiter bevorzugt zwischen 200 nm und 5 µm und besonders bevorzugt zwischen 300 nm und 2,5 µm. Ein weiterer Aspekt der Erfindung ist ein Polymerrohr hergestellt durch Aufwickeln von mindestens einem Polymerfaden, wobei der Polymerfaden einen Durchmesser im Bereich von 50 nm bis 50 µm aufweist.

Die erfindungsgemäßen Polymerrohre weisen bevorzugt einen Innendurchmesser von 0,25 - 20 mm, weiter bevorzugt 0,5 - 15 mm, noch weiter bevorzugt von 1 - 10 mm und besonders bevorzugt von 1,5 - 5 mm auf. Beispielsweise werden für Stents der koronaren Gefäße typischerweise Rohre mit einem Innendurchmesser von 2,0 bis 4,0 mm verwandt mit Wandstärke von 100 bis 200 µm. Entsprechend betragen die Außendurchmesser der Polymerrohre 2,2 bis 4,4 mm.

Das Rohr und der entsprechende Faden sind aus polymerem Material und vorzugsweise aus einem resorbierbaren Polymer hergestellt. Die Polymerrohre besitzen bevorzugt einen kreisrunden Querschnitt. Der Begriff "Wand" oder "Rohrwand", wie hierin verwendet, bezeichnet die Mantel- bzw. Zylinderfläche des Polymerrohrs und somit den durch Aufwickeln mindestens eines Polymerfadens erhaltenen Anteil des Polymerrohrs ohne eventuell zusätzlich aufgebrachte Beschichtungen. Ein erfindungsgemäßes Polymerrohr weist nur eine nahtlose Wand auf. Der Begriff "Wanddicke" oder auch "Wandstärke", wie hierin verwendet, bezeichnet die Differenz der inneren und der äußeren Abmessungen, also des Außen- und des Innendurchmessers des Rohres.

Der Begriff "resorbierbar" oder "bioresorbierbar" wie hierin verwendet bedeutet, dass das Polymer, respektive das Polymerrohr, oder der daraus hergestellte Stent, sich über eine gewisse Zeit in einem menschlichen oder tierischen Organismus langsam auflöst und irgendwann nur noch dessen Abbauprodukte im Körper vorliegen und vom Körper ausgeschieden werden. Zu diesem Zeitpunkt sind feste Bestandteile oder Fragmente des Polymers nicht mehr vorhanden. Die Abbauprodukte sollten physiologisch weitgehend unbedenklich sein.

Erfindungsgemäß ist es bevorzugt, wenn das Polymerrohr hergestellt ist aus mindestens einem bioresorbierbaren Polymer ausgewählt aus der folgenden Gruppe bestehend aus oder umfassend:
Poly(ε-caprolacton), Polyurethan, Polyhydroxybutyrate, Polylactonsäure, Polyglycolsäure, Polylactide, Polyglycolide, Copolymere der Polylactide und Polyglycolide.

Ein weiterer Aspekt der Erfindung bezieht sich auf Polymerrohre hergestellt aus mindestens einem Polymerfaden, wobei der Polymerfaden einen Durchmesser vorzugsweise im Bereich von 50 nm bis 50 µm aufweist und die Polymerfäden aus unterschiedlichen, vorzugsweise bioresorbierbaren Polymeren bestehen. Ein weiterer Aspekt der Erfindung bezieht sich auf Polymerrohre hergestellt aus mehreren Polymerfäden, wobei die Polymerfäden einen Durchmesser vorzugsweise im Bereich von 50 nm bis 50 µm aufweisen und die Polymerfäden aus unterschiedlichen, vorzugsweise bioresorbierbaren Polymeren bestehen. Die erfindungsgemäße Herstellung der Polymerrohre durch Aufwicklung von Polymerfäden lässt die Kombination von unterschiedlichen Polymeren oder Copolymeren in den Polymerrohren zu (im weiteren Hybrid-Rohre genannt), wobei die unterschiedlichen Polymere oder Copolymere in einer gezielten und für das Endprodukt vorteilhaften Anordnung vorliegen können. Dadurch können dem erfindungsgemäßen Polymerrohr gezielt Eigenschaften verschiedener Polymere oder Copolymere verliehen werden. Mit der Wahl der unterschiedlichen Polymere sind daher Hybridrohre herstellbar, die genau auf die Verwendung abgestimmt werden. Erfindungsgemäß bevorzugt ist die Verwendung zur Herstellung von Stents oder anderer Gefäßprothesen. In diesem Fall können die Polymerrohre genau auf die physiologischen Eigenschaften der humanen Gefäße bzw. die Heilungsprozesse und Degradationsvorgänge abgestimmt werden.

So können die Rohre z.B. lagenweise aus unterschiedlichen Polymeren bestehen. Die innere (dem Lumen zugewandte) und äußere (der Gefäßwand zugewandte) Schicht der Wand eines erfindungsgemäßen Polymerrohres kann bevorzugt aus einem Polymer mit sehr langsamer Degradation bestehen, während dazwischen im Inneren der Wand (im Inneren des Rohrquerschnittes) ein Polymer mit hoher Festigkeit (aber gegebenenfalls schneller Degradation) verwendet wird.

Neben der lagenweisen Kombination unterschiedlicher Polymere in der Wand der Polymerrohre (radiale Polymerkombinationen) sind erfindungsgemäß auch Polymerrohre herstellbar, die über die Rohrlänge unterschiedliche Polymere und damit unterschiedliche Eigenschaften aufweisen (axiale Polymerkombinationen). Das heißt die unterschiedlichen Polymere können sowohl in Schichten als auch in Ringen in der Wand des Polymerrohrs variieren bzw. angeordnet sein.

Jegliche Kombinationen von unterschiedlichen Polymeren in radialer und axialer Richtung sind möglich. Dabei entspricht es einer Ausführungsform, dass die Polymerrohre aus einem Polymerfaden hergestellt werden und der Polymerfaden aus unterschiedlichen Polymeren besteht. Die Erfindung umfasst aber auch Polymerrohre, hergestellt aus mindestens zwei Polymerfäden bestehend aus unterschiedlichen Polymeren, bevorzugt aus resorbierbaren Polymeren.

Es ist des Weiteren bevorzugt, wenn die erfindungsgemäßen Polymerrohre eine Wandstärke ≤ 250 µm (z.B. für periphere Gefäße), weiter bevorzugt ≤ 200 µm, weiter bevorzugt ≤ 150 µm (z.B. für koronare Gefäße), und besonders bevorzugt ≤ 100 µm aufweisen. Ein weiterer Aspekt der Erfindung betrifft Polymerrohre, die über die Rohrlänge zu unterschiedlichen Wandstärken aufgewickelt werden. Diese Wandstärken sollen den Festigkeitsbeanspruchungen der, aus den erfindungsgemäßen Polymerrohren hergestellten, Bauteilen entsprechen.

Die erfindungsgemäßen Polymerrohre weisen mindestens 5, bevorzugt mindestens 10 und noch weiter bevorzugt mindesten 20 Schichten bzw. Lagen von Polymerfäden auf. Dabei besteht eine Schicht aus den Wicklungen des mindestens einen Polymerfadens die in einer Ebene verlaufen, die parallel zur Achse des Polymerrohrs liegt, d. h. die Dicke einer Schicht entspricht dem Durchmesser des verwendeten Polymerfadens.

Eine bevorzugte Ausführungsform der Erfindung betrifft ein Polymerrohr, welches mindestens einen Wirkstoff umfasst. Bevorzugt ist dabei, dass sich der mindestens eine Wirkstoff in der Wand des Polymerrohrs befindet. Es handelt sich dabei bevorzugt um Wirkstoffe, die zur Verminderung einer Restenose geeignet sind. Geeignete Wirkstoffe sind insbesondere antiproliferative oder antirestenotische Wirkstoffe.

Der mindestens eine eingesetzte antiproliferative oder antirestenotische Wirkstoff ist bevorzugt ausgewählt aus der Gruppe umfassend oder bestehend aus: Paclitaxel, Rapamycin und deren Derivaten, wie 6-α-Hydroxy-Paclitaxel, Baccatin oder andere Taxotere, Biolimus A9, Myolimus, Novolimus, Pimecrolimus, Tacroliums, Temsirolimus, Zotarolimus, Everolimus, Ridaforolimus oder weiteren sogenannten "Limus"-Derivate (auch Rapalogs genannt).

Es können auch Wirkstoffkombinationen verwendet werden. Dabei kann die Konzentration der Wirkstoffe an verschiedenen Stellen der erfindungsgemäß hergestellten Polymerrohre variieren, so kann an den Enden des Rohrs mehr Wirkstoff enthalten sein als im mittleren Bereich. Die Wirkstoffkonzentration kann auch im Verlauf der Wandstärke variieren und zum Beispiel einen Gradienten aufweisen, so dass auf der äußeren Oberfläche (abluminale Oberfläche) des Polymerrohrs mehr Wirkstoff vorliegt als auf der Oberfläche im Inneren des Rohrs (luminale Oberfläche) oder umgekehrt.

Die vorliegende Erfindung betrifft zudem Verfahren zur Herstellung eines Polymerrohrs, bevorzugt eines resorbierbaren Polymerrohrs, umfassend mindestens die folgenden Schritte:
a) Aufwickeln mindestens eines Polymerfadens mit einem Durchmesser von ≤ 50 µm;
b) Verschmelzen des mindestens einen aufgewickelten Polymerfadens.

Der Durchmesser des Polymerfadens, oder auch Polymerstrang, ist dabei ≤ 50 µm und bevorzugt ≤ 30 µm, weiter bevorzugt ≤ 20 µm, weiter bevorzugt ≤ 15 µm, noch weiter bevorzugt ≤ 10 µm und besonders bevorzugt ≤ 2,5 µm. Die erfindungsgemäß verwendeten Polymerfäden sind bevorzugt Rundfasern, das heißt sie weisen einen runden Querschnitt auf, können jedoch auch andere Querschnitte, wie oval oder eckig aufweisen. Auch Polymerfäden mit einem hohlförmigen Querschnitt, d.h. Hohlfasern kommen in Frage.

Erfindungsgemäß ist es bevorzugt, wenn der mindestens eine Polymerfaden in Schritt a) auf einem sich drehenden Dorn aufgewickelt wird. Der Dorn weist bevorzugt einen konstanten Durchmesser auf, kann aber auch konisch oder wellenförmig ausgeformt sein, so dass der Durchmesser des Polymerrohrs bei gleicher Wandstärke über dessen Länge variiert. Alternativ kann über die Länge des Rohrs auch dessen Wandstärke variieren, so dass bei gleichbleibendem Innendurchmesser der Außendurchmesser variiert. Bei erfindungsgemäßen Polymerrohren, die für die Herstellung von Stents verwendet werden, ist es besonders bevorzugt, wenn zumindest der Innendurchmesser über die gesamte Länge des Polymerrohrs konstant ist.

Der Faden wird bevorzugt mit einer Ablegegeschwindigkeit von mindestens 100 m/min, weiter bevorzugt von mindestens 300 m/min und noch weiter bevorzugt von mindestens 600 m/min auf einem sich rotierenden Dorn abgelegt. Diese hohen Ablegegeschwindigkeiten sind einerseits durch die verfahrensbedingten hohen Geschwindigkeiten der Fadenherstellung vorteilhaft und andererseits erwünscht, um bei den sehr dünnen Fäden die Wicklungszeiten für ein Rohr gering zu halten. Für die bevorzugten Ablegegeschwindigkeiten des Fadens ergeben sich entsprechende, bevorzugte Rotationsgeschwindigkeiten des Dorns von mindestens 10.000 U/min, weiter bevorzugt mindestens 30.000 U/min, noch weiter bevorzugt mindestens 50.000 U/min und besonders bevorzugt mehr als 65.000 U/min.

Es ergibt sich für die Herstellung eines Polymerrohrs ein bevorzugtes Verfahren, welches die folgenden Schritte umfasst:
a) Aufwickeln mindestens eines Polymerfadens mit einem Durchmesser von ≤ 50 µm auf einen sich drehenden Dorn zu einem Polymerrohr; und
b) Verschmelzen des mindestens einen aufgewickelten Polymerfadens.

Es ist zudem bevorzugt, wenn der Polymerfaden, welcher erfindungsgemäß zu Polymerrohren aufgewickelt wird, durch Elektrospinnen oder Schmelzspinnen hergestellt wird. Besonders bevorzugt sind erfindungsgemäße Verfahren, wobei der mindestens eine Polymerfaden durch Elektrospinnen oder Schmelzspinnen hergestellt und gleichzeitig auf einem sich drehenden Dorn aufgewickelt wird. Es ist auch bevorzugt, wenn es sich bei dem verwendeten Polymerfaden um einen unendlich fortlaufenden Faden oder Strang handelt. Dieser unendlich fortlaufende Polymerfaden wird nach Erreichen der gewünschten Wandstärke abgeschnitten.

Beim Elektrospinnen werden sehr dünne Fasern oder Fäden aus Polymerlösungen durch die Behandlung in einem elektrischen Feld hergestellt. Hierbei wird die Polymerlösung an einer Elektrode dosiert und durch das elektrische Feld von der Elektrode abgezogen und beschleunigt. Das Lösungsmittel verdampft bei dem extrem dünnen Faden sehr schnell und es kommt zu einer Ordnung der Polymerketten zu Kristalliten, d.h. die Molekülketten liegen vorzugsweise parallel nebeneinander in Fadenrichtung orientiert (Polymerkristallisation aus der Lösung).

Auf dem sich drehenden Dorn wird dabei der vorzugsweise unendlich fortlaufende Polymerfaden, bevorzugt aus resorbierbaren Polymeren, durch eine Apparatur gleichmäßig bis zu einer vorbestimmten Wandstärke aufgewickelt. Man erhält ein aus einem Polymerfaden gewickeltes Rohr, wobei der Faden bzw. die Fäden idealer Weise perfekt parallel zueinander liegen. Dabei ist es besonders bevorzugt, dass der mindestens eine Polymerfaden so aufgewickelt wird, dass eine Mantelfläche bzw. Wand des Polymerrohrs entsteht, die lückenlos bzw. durchgehend ist. Das erfindungsgemäße Polymerrohr weist daher bevorzugt keine Unterbrechung auf.

Die Poren zwischen den Fäden werden durch anschließendes Verschmelzen der Fäden geschlossen. Das Verschmelzen der Fäden kann so erfolgen, dass die Ausrichtung der Molekülketten nicht verloren geht. Es ist besonders bevorzugt, wenn das Verschmelzen des mindestens einen aufgewickelten Polymerfadens unter Vakuum stattfindet.

Damit liegen die erfindungsgemäß hergestellten Polymerrohre bereits in kristalliner oder teilkristalliner Form vor, ohne dass ein Verstrecken erforderlich ist. Die Kristallinität wird sowohl durch Ausrichtung der Moleküle in den Polymerfäden als auch durch die gerichtete Aufwicklung des Polymerfadens / der Polymerfäden erzeugt. Eine bevorzugte Ausführungsform umfasst daher ein Polymerrohr hergestellt aus mindestens einem aufgewickelten Polymerfaden wobei der Polymerfaden einen Durchmesser im Bereich von 50 nm bis 50 µm aufweist und die einzelnen Wicklungen des Polymerfadens, zumindest in vordefinierten Bereichen, parallel zueinander liegen. Diese Bereiche können z.B. einzelne Schichten von Wicklungen sein, aber auch Bereiche des Polymerrohrs aus denen später eine Strebe eines Stents entsteht und in denen die Richtung des Fadens bzw. der Winkel der Wicklung der späteren Beanspruchung der Strebe entspricht.

Der Begriff "Wicklung", wie hierein verwendet, bezeichnet dabei eine um eine Achse verlaufende Aufwicklung mindestens eines Polymerfadens. Mehrlagige Wicklungen bestehen aus konzentrischen Wendeln, die übereinander liegen. Alle Wicklungen des mindestens einen Polymerfadens, die in einer Ebene parallel zur Achse des Polymerrohrs verlaufen, bilden eine Schicht in der Wand des Polymerrohrs.

Das Verschmelzen der gewickelten Polymerfäden kann erfindungsgemäß wie folgt durchgeführt werden:
Die in Schritt a) aufgewickelten Polymerfäden können noch auf dem Dorn unter erhöhter Temperatur und additional unter Druck von außen zur Schließung der Poren verschmolzen werden. Der Druck kann z.B. durch eine außen anliegende Walze erzeugt werden.

Das Verschmelzen der Polymerfäden erfolgt erfindungsgemäß bevorzugt in einer Vakuumkammer. Vor dem Verschmelzen wird die Kammer evakuiert. Dadurch wird die Luft aus den noch vorhandenen minimalen Zwischenräumen der eng beieinander liegenden Fäden eliminiert und das Verschmelzen erzeugt ein besonders porenfreies Rohr.

Eine andere Methode ist das Aufdehnen des in Schritt a) aufgewickelten Polymerrohres unter Temperatur und Druck von innen, wobei neben dem Verschmelzen die Fäden gleichzeitig gereckt werden. Dieses Verfahren ist der Ballonherstellung (dem Ballonblasen) bei der Ballonkatheterherstellung ähnlich.

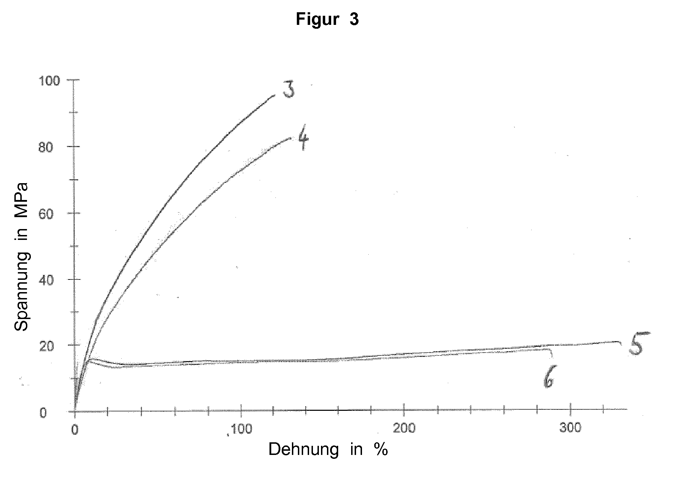

Es konnte überraschend gezeigt werden, dass die Zugfestigkeiten eines erfindungsgemäß hergestellten Polymerrohrs eine hohe Richtungsabhängigkeit zeigen (vgl. Fig. 3). Es wird vermutet, dass dies auf die unidirektional ausgerichteten Polymermoleküle zurückzuführen ist. Besonders vorteilhaft ist, dass zudem die Bruchdehnung in Richtung der Molekülketten trotz der hohen Zugfestigkeit mit über 100 % bei den erfindungsgemäßen Polymerrohren sehr hoch ist, d.h. die Verspannungen (innere Spannungen) des Materials sind gering. Dies ist ein grundsätzlicher, entscheidender Vorteil zu den durch Verstrecken verfestigten Rohren aus dem Stand der Technik, bei denen die Bruchdehnung für hohe Zugfestigkeiten vermindert ist.

Dennoch lässt sich auch bei einem erfindungsgemäß hergestellten Polymerrohr die erhöhte Festigkeit durch Verstrecken, d.h. Dehnen bzw. Verfestigen des Polymerrohrs weiter steigern. Bevorzugt sind dabei folgende Vorgehensweisen:
- Bereits beim Aufwickeln auf den Dorn können die Polymerfäden (mit den bereits ausgerichteten Molekülketten) gereckt werden.
- Eine Verfestigung kann durch Aufdehnen der gewickelten, aber noch nicht verschmolzenen Rohre erfolgen.
- Eine additionale Kombination aus beiden Verfahren (Recken des Fadens beim Aufwickeln und zusätzlich anschließendes Verstrecken des Rohres) ist möglich.
- Es ist möglich, das Verschmelzen der Fäden mit dem Verstrecken des gewickelten Rohres in einem thermomechanischen Vorgang zu kombinieren.
- Das fertig hergestellte Rohr (gewickelt und verschmolzen) lässt sich durch einen anschließenden Dehnvorgang verstrecken.

Ein bevorzugtes, erfindungsgemäßes Verfahren zur Herstellung eines Polymerrohrs umfasst somit, dass der mindestens eine Polymerfaden in Schritt a) beim Aufwickeln auf den sich drehenden Dorn gereckt und damit verfestigt wird.

Ein weiteres bevorzugtes, erfindungsgemäßes Verfahren zur Herstellung eines Polymerrohrs ergibt sich dadurch, dass das Polymerrohr vor Schritt b), während des Verschmelzens gemäß Schritt b) oder nach Schritt b), auf einen Nenndurchmesser aufgedehnt und dabei verfestigt wird.

Daher ergeben sich die folgenden fünf, im Sinne der Erfindung bevorzugten Verfahren zur Herstellung eines Polymerrohrs:
Verfahren zur Herstellung eines Polymerrohrs, welches die folgenden Schritte umfasst:
   a) Aufwickeln mindestens eines Polymerfadens mit einem Durchmesser von ≤ 50 µm auf einen sich drehenden Dorn zu einem Polymerrohr; und beim Aufwickeln recken des mindestens einen Polymerfadens
   b) Verschmelzen des mindestens einen aufgewickelten Polymerfadens.

Beim Recken setzt man beispielsweise die Polymerfäden zum Verformen unter Zugspannung, so dass sich die ungeordneten Polymere und die teilkristallinen Bereiche parallel zur Zugrichtung ausrichten.

Verfahren zur Herstellung eines Polymerrohrs, welches die folgenden Schritte umfasst:
a) Aufwickeln mindestens eines Polymerfadens mit einem Durchmesser von 50 µm zu einem Polymerrohr;
a') Verfestigung durch Aufdehnen des Polymerrohrs;
b) Verschmelzen des mindestens einen aufgewickelten Polymerfadens.

Verfahren zur Herstellung eines Polymerrohrs, welches die folgenden Schritte umfasst:
a) Aufwickeln mindestens eines Polymerfadens mit einem Durchmesser von ≤ 50 µm auf einen sich drehenden Dorn zu einem Polymerrohr; und beim Aufwickeln Recken des mindestens einen Polymerfadens;
a') Verfestigung durch Aufdehnen des Polymerrohrs;
b) Verschmelzen des mindestens einen aufgewickelten Polymerfadens.

Verfahren zur Herstellung eines Polymerrohrs, welches die folgenden Schritte umfasst:
a) Aufwickeln mindestens eines Polymerfadens mit einem Durchmesser von ≤ 50 µm zu einem Polymerrohr;
b) Verschmelzen des mindestens einen aufgewickelten Polymerfadens und parallel zum Verschmelzen Verstrecken des Polymerrohrs.

Verfahren zur Herstellung eines Polymerrohrs, welches die folgenden Schritte umfasst:
a) Aufwickeln mindestens eines Polymerfadens mit einem Durchmesser von ≤ 50 µm zu einem Polymerrohr;
b) Verschmelzen des mindestens einen aufgewickelten Polymerfadens;
b') Verstrecken des verschmolzenen Polymerrohrs durch Dehnung.

Das Verstrecken des erfindungsgemäß hergestellten Polymerrohrs kann bei Raumtemperatur vorzugsweise aber bei erhöhter Temperatur erfolgen.

Zur Einstellung und/oder Fixierung der mechanischen Eigenschaften der erfindungsgemäßen Polymerrohre, insbesondere wenn ein Verstrecken stattgefunden hat, kann noch eine thermische Nachbehandlung durchgeführt werden. Durch Tempern bei Temperaturen unterhalb des Schmelzpunktes wird die Kristallinität erhöht. Durch Abschrecken können diese kristallinen Strukturen, aber auch die amorphe Strukturen fixiert werden.

Überraschend wurde gefunden, dass Polymerrohre, hergestellt durch Aufwicklung möglichst dünner Polymerfäden, z.B. auf sich drehenden Dornen, eine erhöhte Festigkeit bei gleichzeitig optimaler Bruchdehnung aufweisen. In den dünnen Fäden mit Durchmessern im Mikrometerbereich oder Submikrometerbereich (mit einem Durchmesser im Bereich von 50 nm bis 50 µm) sind die Moleküle bereits weitgehend unidirektional ausgerichtet.

Wie erläutert, führen die derzeitigen festigkeitssteigernden Methoden, angewendet bei Polymerrohren aus dem Stand der Technik, zu starken Einschränkungen bei der Verwendung als Ausgangsmaterial für Polymerstents. Die erfindungsgemäße Herstellung von Polymer-Rohren beseitigt die starken Einschränkungen bzw. verbessert die Eigenschaften der Rohre, was insbesondere für deren Verwendung zur Herstellung von Stents und anderen Gefäßimplantaten entscheidend ist.

Beim Verstrecken (Dehnen) von Polymerrohren laufen grundsätzlich zwei Effekte parallel ab:
A) Die einzelnen langkettigen Moleküle werden in Dehnungsrichtung entknäuelt und gestreckt. In Richtung der Molekülketten ist die Festigkeit erhöht und quer zur Molekülrichtung geringer.
B) Beim Dehnen bewegen sich die Molekülketten aber auch zueinander, gleiten aufeinander ab und werden zueinander verspannt (Verfestigung). Es entstehen innere Spannungen. Mit zunehmenden inneren Spannungen nimmt die Dehnbarkeit des Werkstoffes ab.

Das Verstrecken ist daher nur begrenzt möglich, da Polymerrohre nur begrenzt ohne Bruchinduzierung gedehnt werden können. Zum Einen lassen sich die Moleküle nicht beliebig strecken und zum Anderen dürfen die inneren Spannungen die Bruchfestigkeit nicht überschreiten.

Erfindungsgemäß sollen die Vorgänge der Molekülausrichtung und der Verfestigung während der erfindungsgemäßen Herstellung der Polymerrohre völlig, zumindest aber weitgehend, getrennt voneinander ablaufen, um für beide Vorgänge ein Optimum zu erreichen.

Die Molekülausrichtung wird schon beim ersten Schritt der Herstellung der Polymerrohre, nämlich beim Aufwickeln der Polymerfäden gewährleistet. Die Rohrherstellung erfolgt durch Aufwicklung möglichst dünner Polymerfäden, bevorzugt auf schnelldrehenden Dornen. In den dünnen Fäden mit Durchmessern im Mikrometerbereich (≤ 50 µm) oder Submikrometerbereich (≤ 1 µm) sind die Moleküle bereits weitgehend unidirektional ausgerichtet. Die Fadenherstellung kann z.B. durch Extrusion aus kleinsten Spinndüsen (Schmelzspinnen) erfolgen oder vorzugsweise durch Elektrospinnen. Eine Dehnung oder Verfestigung findet danach in einem oder mehreren weiteren Schritten statt.

Die starke Richtungsabhängigkeit der Festigkeit der erfindungsgemäß hergestellten Rohre kann man insbesondere für die Herstellung von Gefäßimplantaten, vorzugsweise Stents, nutzen. Unter Stents werden hierbei gitterförmige oder netzförmige Endoprothesen verstanden, die in ein Hohlorgan oder einen Körperhohlraum eingesetzt werden, um diesen offen zu halten. Das Grundgerüst eines Stents, womit hier die polymeren Streben ohne Beschichtung bezeichnet werden, bildet kein massives Rohr, sondern ein Gittergeflecht. Betrachtet man beispielsweise das Grundgerüst eines vaskulären Stents, so wird dieses aus einem massiven Rohr z.B. mittels Laser geschnitten, so dass sich einzelne, möglichst dünne Streben ergeben, welche untereinander verbunden sind. Die Anordnung und Ausformung der Streben und Knotenpunkte wird als Stentdesign bezeichnet. Im Sinne der vorliegenden Erfindung können alle üblichen Stentgeometrien verwendet werden.

Stents oder Gefäßstützen benötigen eine hohe radiale Festigkeit, um das Gefäß nach der Dilatation (Aufdehnung) offen zu halten. Hingegen ist in axialer Richtung, d.h. in Längsrichtung der Stents keine hohe Festigkeit gefordert. In radialer Richtung (Umfangsrichtung) ist zudem eine hohe Bruchdehnung gefordert, um den Stent bei Implantation mittels Ballonkatheter ausreichend und ohne Bruchgefahr dehnen zu können. Die erfindungsgemäß hergestellten Polymerrohre weisen genau diese Eigenschaften auf. Sie zeichnen sich durch eine hohe Festigkeit bei gleichzeitiger, hoher Bruchdehnung in radialer Richtung, d.h. in Umfangsrichtung, aus.

Ein bevorzugter Aspekt der vorliegenden Erfindung ist es daher Polymerrohre zur Verfügung zu stellen, die zur Herstellung eines Stents besonders gut geeignet sind. Diese Polymerrohre, vorzugsweise resorbierbaren Polymerrohre, können nach einem der hierin beschriebenen, erfindungsgemäßen Verfahren hergestellt werden. Des Weiteren umfasst die vorliegende Erfindung daher einen Stent oder ein Gefäßimplantat, bevorzugt einen resorbierbaren Stent, hergestellt aus einem der hierin offenbarten Polymerrohre. Es handelt sich bei dem resorbierbaren Stent bevorzugt um einen Stent für Blutgefäße, Harnwege, Atemwege, Gallenwege oder den Verdauungstrakt. Unter diesen Stents sind wiederum die Stents für Blutgefäße oder allgemeiner für das Herz-Kreislaufsystem bevorzugt.

Beim Schneiden eines Stents werden Flächen zwischen den einzelnen Streben herausgeschnitten. Ein Stent oder ein vaskuläres Implantat weist daher eine Vielzahl von massiven Gerüstkomponenten (z.B. Streben in Form von Ringen, Spiralen, Wellen und Drähten) auf, sowie eine Vielzahl von Zwischenräumen zwischen diesen massiven Komponenten. Bei der gängigen Ausführungsform von Endoprothesen oder Stents laufen die Streben in Knotenpunkten zusammen. Es gibt jedoch auch Ausführungsformen von Endoprothesen, bei denen keine oder fast keine Knotenpunkte vorhanden sind und die Streben z.B. die Form von Ringen oder Spiralen haben. Bevorzugt handelt es sich um ballonexpandierbare Stents, welche mittels Katheter bis zur erkrankten oder zu behandelnden Stelle geschoben werden, wo die Stents auf ihren definierten Normdurchmesser aufgeweitet werden.

Ferner ist ein Kriechen des Materials, d.h. der gecrimpten bzw. im Gefäß dilatierten Stents unerwünscht, was durch eine Begrenzung der Dehnung bzw. der Verstreckung erzielt werden kann. Um unerwünschte Kriechvorgänge der implantierten Stents weitgehend zu vermeiden, hat es sich erfindungsgemäß als vorteilhaft erwiesen, Stents z.B. durch Laserschneiden im gedehnten Zustand herzustellen. Das bedeutet, die Stents werden aus erfindungsgemäßen Polymerrohren hergestellt, deren Durchmesser dem Nenndurchmesser, also dem Innendurchmesser des Stents im aufgeweiteten Zustand, entspricht. Danach kann der erfindungsgemäße Stent dennoch ohne Schwierigkeiten auf einen Katheterballon aufgebracht (gecrimpt) werden. Die Erfindung betrifft damit Stents hergestellt aus den erfindungsgemäßen Polymerrohren, wobei der Innendurchmesser des Polymerrohrs (des nicht aufgedehnten Polymerrohrs) dem Innendurchmesser des Stents nach Implantation entspricht.

Die erfindungsgemäßen Polymerrohre zur Herstellung eines Stents weisen daher vorzugsweise Innendurchmesser auf, die den aufgedehnten Stents entsprechen. Es ist bevorzugt, wenn das erfindungsgemäße Polymerrohr einen Innendurchmesser von 0,5 - 15 mm, weiter bevorzugt von 1 - 10 mm und noch weiter bevorzugt von 2 - 6 mm aufweist. Aus Polymerrohren dieser Durchmesser, die den Durchmessern dilatierter Stents entsprechen (Nenndurchmesser oder Nominaldurchmesser), werden die Gefäßimplantate bevorzugt lasergeschnitten. Damit liegen die hergestellten Stents im aufgedehnten (dilatierten) Zustand vor.

Außerdem ist dies vorteilhaft, da im aufgedehnten Zustand die Stentstege oder - streben vorzugsweise in Umfangsrichtung weisen und damit in Richtung der aufgewickelten Polymerfäden respektive in Richtung der Molekülketten bzw. der hohen Festigkeiten. Anschließend werden die Stents auf den Ballonkatheter gecrimpt, um sie implantieren zu können.

Die vorliegende Erfindung betrifft somit Verfahren zur Herstellung eines Stents, bevorzugt eines resorbierbaren Stents, umfassend mindestens die folgenden Schritte:
a) Aufwickeln mindestens eines Polymerfadens mit einem Durchmesser von ≤ 50 µm zu einem Polymerrohr;
b) Verschmelzen des mindestens einen aufgewickelten Polymerfadens;
c) Schneiden eines Stents aus dem Polymerrohr, vorzugsweise mittels Laser.

Ein bevorzugtes Verfahren der vorliegenden Erfindung ist ein Verfahren zur Herstellung eines Stents, bevorzugt eines resorbierbaren Stents, umfassend mindestens die folgenden Schritte:
a) Aufwickeln mindestens eines Polymerfadens mit einem Durchmesser von ≤ 50 µm zu einem Polymerrohr;
b) Verschmelzen des mindestens einen aufgewickelten Polymerfadens;
c) Schneiden eines Stents aus dem Polymerrohr, vorzugsweise mittels Laser, wobei der Durchmesser des Polymerrohrs dem Nenndurchmesser des Stents entspricht.

Die vorliegende Erfindung betrifft auch Stents erhältlich nach einem der erfindungsgemäßen Herstellungsverfahren. Somit bezieht sich die vorliegende Erfindung auch auf Stents, deren Stentstreben aus verschmolzenen Teilstücken aus mindestens einem Polymerfaden bestehen, wobei der Polymerfaden einen Durchmesser von ≤ 50 µm aufweist. Die Teilstücke liegen dabei bevorzugt mehrlagig (mindestens 5 Lagen) und/oder parallel zueinander vor.
Die vorliegende Erfindung bezieht sich insbesondere auf Stents, welche durch Laserschneiden aus den erfindungsgemäßen Polymerrohren hergestellt wurden. Das Stentgerüst, d.h. die Stentstreben und Knotenpunkte, dieser Stents bestehen aus gerichteten und miteinander verschmolzenen Fadenstücke. Der Begriff "gerichtet" bedeutet in diesem Zusammenhang, dass die Fadenstücke vorzugsweise parallel zueinander ausgerichtet sind. Betrachtet man, dass das Polymerrohr aus wenigen Fäden oder vorzugsweise sogar nur einem Faden besteht und die Zwischenräume zwischen den Stentstreben mittels Laser aus dem Polymerrohr herausgeschnitten worden sind, so ist ersichtlich, dass die Stentstreben aus einer Vielzahl von Fadenstücken bestehen, welche sich ursprünglich durch die Zwischenräume der Stentstreben erstrecken und sich in einer angrenzenden Stentstrebe fortsetzen. Da die Aufwicklung des Polymerfadens oder der Polymerfäden zu dem Polymerrohr nicht willkürlich oder nach dem Zufallsprinzip ungeordnet übereinander, sondern in geordneter und vorzugsweise paralleler Weise erfolgt, bestehen nach dem Laserschneiden die Polymerstents aus Stentstreben und Knotenpunkten (sowie vorhanden), welche aus geordneten Fadenstücken aufgebaut sind. Die Fadenstücke verlaufen dabei in der Regel nicht entlang der Längsachse durch die Stentstrebe und auch in der Regel nicht senkrecht dazu, sondern quer durch die jeweiligen Stentstreben. Bevorzugt verlaufen die Fadenstücke in mindestens 90% der Stentstreben in einem Winkel von 5° bis 85°, bevorzugt 10° bis 80° bezogen auf die jeweilige Längsachse durch die jeweilige Stentstrebe. Der erfindungsgemäße Polymerstent weist somit Stentstreben aus Fadenstücken von Polymerfäden auf, wobei das einzelne Fadenstück durch die Stentstrebe verläuft. Verzugsweise verlaufen die einzelnen Fadenstücke ohne Unterbrechung durch die jeweilige Stentstrebe. Bevorzugt verlaufen die einzelnen Fadenstücke geradlinig durch die jeweilige Stentstrebe. Zudem sind die einzelnen Fadenstücke, welche durch eine Stentstrebe verlaufen vorzugsweise parallel zueinander. Der Verlauf der einzelnen Fadenstücke durch die jeweilige Stentstrebe erfolgt vorzugsweise unter einem Winkel von 5° bis 85°, weiter bevorzugt 10° bis 80° bezogen auf die Längsachse durch die jeweilige Stentstrebe. Zudem sind die einzelnen Fadenstücke, welche durch eine Stentstrebe oder durch einen Knotenpunkt verlaufen miteinander verschmolzen und zudem vorzugsweise auch gerichtet. Ein besondere Merkmal ist zudem, dass sich die Fadenstücke nach dem Zwischenraum zwischen zwei angrenzenden Stentstreben in einer angrenzenden Stentstrebe fortsetzen. Dieser besondere Aufbau ist dadurch bedingt, dass die Stents aus einem Polymerrohr bestehend aus geordnet aufgewickelten Polymerfäden geschnitten wurden. Die Ausrichtung der Fadenstücke in einer Stentstrebe setzt sich daher gemäß der Aufwicklung des Polymerrohres in einer benachbarten Stentstrebe fort. Dieser besondere Aufbau und die dadurch erhaltenen physikalischen und chemischen Eigenschaften sind einzigartig für die erfindungsgemäßen Polymerstents. Innendurchmesser, Wanddicke bzw. Strebendicke und Dicke der einzelnen Fadenstücke des Polymerstents entsprechen denen des Polymerrohres. Nur die Fadenlänge in dem Polymerstent ist aufgrund des Herausschneidens der Strebenzwischenräume drastisch reduziert. Die durchschnittliche Länge eines Fadenstücks beträgt vorzugsweise weniger als 90% der Länge der jeweiligen Stentstrebe, wenn als Länge einer Stentstrebe der Abstand von einem Knotenpunkt zum nächsten Knotenpunkt bezeichnet wird und vorzugsweise mehr als der doppelte Durchmesser der jeweiligen Stentstrebe.

Dabei treffen alle hierin gemachten Aussagen zu bevorzugten Ausführungsformen eines erfindungsgemäßen Verfahrens zur Herstellung eines Polymerrohrs entsprechend auch auf die Verfahren zur Herstellung des Stents zu.

Dieses Verfahren der Stentherstellung im gedehnten Zustand weist den weiteren Vorteil auf, dass unerwünschte Kriechvorgänge der Polymere weitgehend vermieden werden. Verformte Polymere neigen zum Kriechen in ihre Ursprungsform. Ein Stent, der im ungedehnten (gecrimpten) Zustand hergestellt und anschließend gedehnt wird, neigt zum Kriechen in Richtung seines ursprünglichen, kleineren Durchmessers. Dies ist für eine Stentimplantation nachteilig, da der implantierte Stent seinen Durchmesser im Gefäß wieder verringert. Dieses Verhalten wird bei den derzeitig zugelassenen, bioresorbierbaren Stents beobachtet.

Wird der Stent hingegen aus einem Polymerrohr mit einem großen Rohrdurchmesser, der dem dilatierten Gefäßdurchmesser entspricht, geschnitten, so wird er diesen Durchmesser nach der Dilatation im Gefäß beibehalten. Es ist erfindungsgemäß bevorzugt, wenn der Stent aus einem erfindungsgemäßen Polymerrohr mittels Laser ausgeschnitten wird. Dabei ist es weiter bevorzugt, wenn das erfindungsgemäße Polymerrohr dabei einen Durchmesser aufweist, welcher dem Durchmesser des aufgedehnten (bzw. aufgeweiteten oder inflatierten) Stents entspricht. Dabei entspricht der Innendurchmesser des aufgedehnten Stents dem Innendurchmesser des erfindungsgemäßen Polymerrohrs, welches zu seiner Herstellung verwendet wird. Das gleiche gilt für die Außendurchmesser, es sei denn der Stent wird nach der Herstellung aus dem Polymerrohr noch mit einer Beschichtung, z.B. einer wirkstofffreisetzenden Beschichtung, versehen.

Ein weiterer Vorteil der Herstellung der Stents aus Rohren deren Durchmesser dem aufgedehnten Durchmesser, d.h. Nenndurchmesser, des Stents entspricht ist, dass die Bruchgefahr bei einem Überdehnen über den Nenndurchmesser unkritischer ist als bei Stents, die aus kleineren Rohrdurchmessern (zumeist ein Durchmesser der nahezu dem des gecrimpten, deflatierten Stents entspricht) hergestellt wurden. Da die Festigkeit der Polymerrohre aus dem Stand der Technik zu Lasten der Dehnbarkeit optimiert wurde, sind die aus kleineren Rohrdurchmessern hergestellten Stents nur sehr begrenzt dehnbar und eine Überdehnung führt schnell zum Bruch. Bei den erfindungsgemäß hergestellten Stents sind bei gleicher, begrenzter Dehnbarkeit größere Gefäßdurchmesser erreichbar.

Es ist zudem möglich, die erfindungsgemäß hergestellten Polymerrohre noch zielgerichteter an die mechanischen Anforderungen von Gefäßimplantaten anzupassen:

Beim Aufwickeln des Polymerfadens wird ein sich schnell drehender Dorn so hin- und her bewegt, dass der Faden z.B. kreuzweise unter einem bestimmten Winkel aufgewickelt wird. Dies kann gezielt im Hinblick auf die späteren Belastungen (Richtungen der Spannungsbeanspruchungen) der einzelnen Stege oder Streben des Stents nach Implantation, z.B. in ein Blutgefäß, geschehen. Die Fäden sollen dabei vorzugsweise in Richtung der höchsten Festigkeitsbeanspruchung liegen. Ferner ist es möglich, den Faden lagenweise d.h. in Schichten mit verschiedenen Winkeln aufzuwickeln und so gewünschte zielgerichtete Festigkeiten zu erreichen. Es ist auch möglich, in axialer Richtung des Rohres unterschiedliche Rohrwandstärken zu realisieren, die den unterschiedlichen Festigkeitsansprüchen eines Stents z.B. in der Mitte und an den Enden Rechnung trägt. Hierzu werden die Wicklungsstärken, d.h. die Anzahl der Wicklungen des Fadens in Längsrichtung auf dem Dorn variiert.

Ein anderes bevorzugtes Verfahren zur Herstellung eines Polymerrohrs gemäß der vorliegenden Erfindung, umfasst einen Schritt a) bei dem der Polymerfaden zielgerichtet lagen- und kreuzweise unter einem definierten Winkel aufgewickelt wird.

Daher ergibt sich für die Herstellung eines Polymerrohrs ein besonders bevorzugtes Verfahren, welches die folgenden Schritte umfasst:
a) Aufwickeln mindestens eines Polymerfadens mit einem Durchmesser von 50 µm auf einen sich drehenden Dorn zu einem Polymerrohr, wobei der Dorn dabei so hin und her bewegt wird, dass der Polymerfaden unter einem bestimmten Winkel kreuzweise aufgewickelt wird;
b) Verschmelzen des mindestens einen aufgewickelten Polymerfadens.

Ein weiteres bevorzugtes Verfahren für die Herstellung eines Polymerrohrs umfasst die folgenden Schritte:
a) Aufwickeln mindestens eines Polymerfadens mit einem Durchmesser von ≤ 50 µm auf einen sich drehenden Dorn zu einem Polymerrohr, wobei der Dorn dabei so hin und her bewegt wird, dass der Polymerfaden gemäß einem vorgegebenen Muster aufgewickelt wird;
b) Verschmelzen des mindestens einen aufgewickelten Polymerfadens.

Dabei ist es bevorzugt, wenn das Muster entsprechend dem Stentdesign gewählt wird und z.B. spiralförmig ist.

Ein weiteres bevorzugtes Verfahren für die Herstellung eines Polymerrohrs umfasst die folgenden Schritte:
a) Aufwickeln mindestens eines Polymerfadens mit einem Durchmesser von ≤ 50 µm zu einem Polymerrohr, wobei der mindestens eine Polymerfaden in mindestens zwei Schichten mit verschiedenen Winkeln aufgewickelt wird;
b) Verschmelzen des mindestens einen aufgewickelten Polymerfadens.

Es ist dabei besonders bevorzugt, wenn die Winkel der Schichten entsprechend der Festigkeitsbeanspruchung der aus den Rohren hergestellten Bauteile gewählt werden. Im Fall eines Stents sind dies vor allem Radialkräfte, die auf die einzelnen Stentstreben einwirken. Daher ist es von besonderem Vorteil, dass die Winkel je nach Stentdesign frei wählbar sind.

Es ist bevorzugt, wenn die Wicklungsstärken, d.h. die Anzahl der übereinanderliegenden Wicklungen bzw. Schichten des Fadens, bei den erfindungsgemäß hergestellten Polymerrohre zwischen 5 Wicklungen und 1000 Wicklungen, weiter bevorzugt zwischen 10 Wicklungen und 600 Wicklungen, weiter bevorzugt zwischen 25 Wicklungen und 500 Wicklungen und noch weiter bevorzugt zwischen 50 Wicklungen und 300 Wicklungen liegt.

Bei einem weiteren bevorzugten Verfahren zur Herstellung eines Polymerrohrs gemäß der vorliegenden Erfindung, werden im Schritt a) beim Aufwickeln der Fäden Wirkstoffe eingebracht. Es handelt sich dabei bevorzugt um Wirkstoffe, die zur Verminderung einer Restenose verwendet werden können. Geeignete Wirkstoffe sind insbesondere antiproliferative oder antirestenotische Wirkstoffe.

Der mindestens eine eingesetzte antiproliferative oder antirestenotische Wirkstoff ist bevorzugt ausgewählt aus der Gruppe umfassend oder bestehend aus: Paclitaxel, Rapamycin und deren Derivaten, wie 6-a-Hydroxy-Paclitaxel, Baccatin, Docetaxel, Biolimus A9, Myolimus, Novolimus, Pimecrolimus, Tacroliums, Temsirolimus, Zotarolimus, Everolimus, Ridaforolimus oder weiteren sogenannten Taxanen, bzw "Limus"-Derivaten.

Es können auch Wirkstoffkombinationen eingebracht werden. Dabei können die Wirkstoffe in unterschiedlichen Konzentrationen vorliegen. Die Konzentration eines verwendeten Wirkstoffs kann ebenfalls zwischen verschiedenen Teilbereichen des erfindungsgemäßen Polymerrohrs und damit des daraus herstellbaren Stents variieren. So kann im Bereich der Enden des Polymerrohrs mehr Wirkstoff enthalten sein, als im mittleren Bereich. Alternativ kann auf der luminalen (blutseitigen) Rohrinnenseite ein anderer Wirkstoff eingebracht werden als auf der abluminalen (gefäßseitigen) Rohraußenseite. Denkbar wären ein Wirkstoff zur Förderung der Endothelialisierung auf der luminalen Rohrinnenseite und ein Wirkstoff zur Proliferationshemmung auf der abluminalen Rohraußenseite.

Beim lagenweisen Aufwickeln von elektro- oder schmelzgesponnenen Polymerfäden können Wirkstoffe zwischen den Polymerfäden aufgebracht werden. Damit ist es möglich, die Wirkstoffe direkt in die Wand des Polymerrohrs zu integrieren. Der Polymerfaden kann z.B. vor dem Aufwickeln mit einer Wirkstofflösung imprägniert werden. Eine besondere Möglichkeit der Einbeziehung von Wirkstoffen ergibt sich beim Elektrospinnen. Beim Elektrospinnen wird das vorzugsweise resorbierbare Polymer in einem Lösungsmittel in der Regel bei Raumtemperatur aufgelöst. In diese Lösung kann der Wirkstoff mit eingebracht werden, sofern er mit dem Lösungsmittel verträglich ist. Damit befindet sich der Wirkstoff bereits in dem elektrogesponnenen Faden, der zu einem Rohr aufgewickelt wird.

Beim anschließenden Verschmelzen (Verbacken) der Polymerrohre sind die Temperaturen deutlich niedriger und damit wirkstoffschonender als beim Extrudieren oder Spritzgießen. Beim Extrudieren bzw. Spritzgießen der Polymerrohre werden die Schmelztemperaturen der Polymere deutlich überschritten.

Auch kann ein Wirkstoffgradient über die Wandstärke des Polymerrohrs vorteilhaft sein. Hierzu können z.B. Polymerfäden mit unterschiedlicher Wirkstoffkonzentration bzw. mit unterschiedlichen Wirkstoffen zur Herstellung des Polymerrohrs verwendet werden. Alternativ können einem kontinuierlichen Polymerfaden bei dessen Herstellung unterschiedliche Wirkstoffkonzentrationen bzw. unterschiedliche Wirkstoffe zugesetzt werden. In Kombination mit dem mindestens einen Wirkstoff oder der Wirkstoffkombination können weitere Substanzen z.B. Transportvermittler eingebracht werden.

Als Lösungsmittel eignen sich bevorzugt organische Lösungsmittel wie beispielsweise Chloroform (Trichlormethan), Methylenchlorid (Dichlormethan), Tetrafluorethylen (TFE), Tetrahydrofuran (THF), Aceton (Dimethylketon), Diethylether, Methanol, Ethanol, Propanol, Isopropanol, Diethylketon, Dimethylformamid (DMF), Dimethylacetamid, Essigsäureethylester, Dimethylsulfoxid (DMSO), Benzol, Toluol, Xylol, t-Butylmethylether (MTBE), Cyclohexan, N-Methyl-2-Pyrrolidone, Pentan, Hexan, Heptan, wobei Methylenchlorid und Chloroform bevorzugt sind.

Die spätere Freisetzung der Wirkstoffe erfolgt mit der Implantation der Polymerrohre bzw. der daraus hergestellten Implantate wie Stents. In der Anfangsphase nach der Implantation von Stents, hergestellt aus den erfindungsgemäßen Polymerrohren, finden Zersetzungsvorgänge der Polymermoleküle statt, ohne dass der Stent degradiert, d.h. ohne Masseverluste. Die Wirkstoffabgabe erfolgt im Wesentlichen durch Diffusion. Bei der später einsetzenden Degradation erfolgt die Wirkstoffabgabe durch Erosion. Möglich ist auch, die Wirkstoffe in unterschiedlichen Konzentrationen in die Rohrwand einzubringen und damit dem Verlauf der Diffusion und der Degradation des Stents und/oder dem Heilungsprozess des Gefäßes Rechnung zu tragen. Im Gegensatz dazu wird bei derzeitig zugelassenen, beschichteten, resorbierbaren Polymerstents erst die Wirkstoffbeschichtung abgebaut und danach der Stent, ohne das noch Wirkstoffe vorhanden sind.

Dennoch ist es auch möglich die erfindungsgemäß hergestellten Polymerrohre bzw. Polymerstents mit einer zusätzlichen Beschichtung zu versehen. Dabei handelt es sich bevorzugt um eine wirkstofffreisetzende Beschichtung. Der Wirkstoff oder eine Wirkstoffkombination kann dabei alleine oder in einer geeigneten Matrix auf die polymere Oberfläche der Rohre oder Stents aufgebracht werden. Eine geeignete Matrix kann ein Polymer, bevorzugt ein resorbierbares Polymer, sein. Das Polymer der wirkstofffreisetzenden Beschichtung kann dabei mit dem Polymer des Grundgerüsts identisch sein.

Mit dem Wirkstoff zusammen können auch weitere Substanzen aufgebracht werden, die dessen Freisetzung bzw. Haftung auf dem Stent beeinflussen. Als pharmakologisch verträgliche Träger in einer wirkstofffreisetzende Beschichtung können Substanzen dienen, ausgewählt aus der Gruppe bestehend aus oder umfassend: Stärke, Natrium-Carboxymethylstärke, Sorbitol, Sucrose, Magnesiumstearat, Dicalciumphosphat, Calciumsulfat, Talk, Mannitol, Polyvinylalkohole, Polyvinylpyrrolidon, Gelatine, natürliche Zucker, sowohl natürliche als auch synthetische Gummis wie beispielsweise Akaziengummi oder Guar-Gummi, Natriumalginat, Natriumbenzoat, Natriumacetat, Glyceride, Isopropylmyristate und - palmitate, Citrate, wie Tributyl- und Triethylcitrate und deren Acetylderivate, Phtalate wie Dimethylphtalat oder Dibutylphtalat, Benzoesäurebenzylester, Triacetin, 2-Pyrrolidon, Agar, Cellulose, Cellulosederivate wie Methylcellulose, NatriumCarboxymethylcellulose und Hydroxypropylmethylcellulose,.

Die wirkstofffreisetzende Beschichtung kann mittels bekannter Verfahren wie beispielsweise Sprühverfahren, Tauchverfahren, Plasmaverfahren, Pinselverfahren, Spritzenverfahren, Elektrospinnen oder Pipettierverfahren auf das Grundgerüst (bestehend aus Stentstreben, die aus dem erfindungsgemäßen Polymerrohr geschnitten wurden) aufgebracht werden.

Aus den obigen Ausführungen wird deutlich, dass die Eigenschaften der erfindungsgemäßen Polymerrohre auch Eigenschaften der aus diesen Rohren hergestellten Stents bzw. Gefäßstützen sind. Somit sollen alle hierin angeführten Merkmale und Merkmalskombinationen der erfindungsgemäßen Polymerrohre auch im Zusammenhang mit den erfindungsgemäßen Stents verstanden werden. Sowohl die Polymerrohre als auch die Stents werden in ihrer Charakteristik vor allem durch die erfindungsgemäßen Herstellungsverfahren geprägt. Die vorliegende Erfindung betrifft demnach auch Polymerrohre und Stents erhältlich nach einem der erfindungsgemäßen Verfahren. Die Polymerrohre und Stents hergestellt durch eins der erfindungsgemäßen Verfahren zeichnen sich durch eine erhöhte radiale Festigkeit bei gleichzeitig hoher oder sogar erhöhter Bruchdehnung aus, wobei die radiale Festigkeit bevorzugt > 80 MPa, weiter bevorzugt > 90 MPa, noch weiter bevorzugt > 100 MPa beträgt und die Bruchdehnung > 30 %, weiter bevorzugt > 40 %, noch weiter bevorzugt > 50 % ist.

### Figurenbeschreibung

- **Figur 1:**: Spannungs-Dehnungs-Diagramm eines PLLA Rohres (1.8 x 0.15 mm) aus dem Stand der Technik (Anmerkung: Die beiden Kurven mit Bruchdehnungen von nur 30 % und 80 % sind eine Folge von Quetschungen direkt an der Einspannung der Proben, also nicht durch das Material bedingt.)
- **Figur 2:**: Spannungs-Dehnungsdiagramm eines radial aufgedehnten PLLA Rohres aus dem Stand der Technik Im Vergleich zu Figur 1 sind deutlich die höhere Festigkeit, aber auch die geringere Bruchdehnung zu erkennen. Die geringere Bruchdehnung führt zu einer begrenzten Dehnbarkeit der Polylactid-Stents hergestellt aus solchen Rohren und zu einer Bruchgefahr bei nur geringer Überdehnung über den Nominaldurchmesser des Stents.
- **Figur 3:**: Richtungsabhängigkeit der Zugfestigkeiten unidirektional ausgerichteter Molekülketten eines erfindungsgemäßen Polymerrohrs aus Polycaprolacton (Kurven 3 und 4: Festigkeit in Molekülrichtung, Kurven 5 und 6: Festigkeit quer zur Molekülausrichtung)

### Beispiele

### Beispiel 1: Herstellung eine erfindungsgemäßen Polymerrohrs

Ein erfindungsgemäßes Polymerrohr wurde hergestellt, indem das hochreine Polymergrundmaterial Polycaprolacton (PCL) in Tetrafluorethylen (TFE) gelöst wird und zwar 170 mg PCL pro 1 ml TFE und im Elektrospin-Verfahren zu einem ca. 2 µm dünnen Faden gezogen wird. Der Faden wird mit hoher Geschwindigkeit von ca. 700 m/min von der Elektrospindüse abgezogen. Das Tetrafluorethylen verdampft augenblicklich aus dem sehr dünnen Faden, der auf einem sich drehenden Dorn aufgewickelt wird. Dabei wird der Dorn hin und her bewegt, um eine gleichmäßige Aufwicklung, d.h. eine gleichmäßige lagenweise Wicklungsstärke zu erzielen.

Anschließend werden die eng nebeneinander liegenden Fäden des Polymerrohres bei einer Temperatur oberhalb der Glas- aber unterhalb der Schmelztemperatur bei ca. 65 °C verschmolzen. Um eine möglichst porenfreie Rohrwandung mit einer hohen Dichte sicherzustellen, wird das Verschmelzen in einer Vakuumkammer durchgeführt und von außen eine mechanische Kraft auf das Rohr ausgeübt, d.h. das Rohr wird auf den Dorn gepresst.

### Beispiel 2: Herstellung eines erfindungsgemäßen Stents

Nach Abkühlung wird das gemäß Beispiel 1 hergestellte Rohr vom Dorn gezogen und in eine Laseranlage eingespannt. Mit einem Ultrakurzpulslaser wird das Stentdesign in das Polymerrohr geschnitten. Die Ausschnitte fallen heraus, so dass nur die Stentstruktur bestehend aus den einzelnen Stentstegen übrig bleiben. Nach Reinigungsschritten ist der Stent fertig, um auf einen Ballonkatheter gecrimpt und anschließend implantiert zu werden.

### Beispiel 3: Herstellung eine erfindungsgemäßen Polymerrohrs enthaltend Paclitaxel

Wenn der Stent Wirkstoffe freisetzen soll, um das Heilungsverhalten zu verbessern, so kann der Wirkstoff beispielsweise direkt bei der Herstellung ins Polymerrohr eingebracht werden.

Hierzu wurde in die Tetrafluorethylenlösung des Polymers aus Beispiel 1 Paclitaxel in zwei verschiedenen Konzentrationen mit hinzu gemischt (1:10 und 1:3 Paclitaxel zu Polymer). Da das Polymerrohr lagenweise über die Rohrwandstärke zwei verschiedene Wirkstoffkonzentrationen enthalten sollte, wird zunächst unter Verwendung der ersten Lösung im Elektrospin-Verfahren ein ca. 0,75 µm dünner Faden gezogen und dieser auf einem mit hoher Geschwindigkeit drehenden Dorn aufgewickelt. Dabei wird der Dorn hin und her bewegt, um eine gleichmäßige Aufwicklung zu erzielen. Nach 200 Lagen wird das Aufwickeln des Fadens unterbrochen und mit einem Faden aus der Lösung mit der zweiten Wirkstoffkonzentration fortgesetzt bis insgesamt 300 Lagen aufgewickelt sind. Das Verfahren der Fadenverschmelzung änderte sich gegenüber Beispiel 1 nicht. Auch das Verfahren des Laserschneidens des Stents wie in Beispiel 2 beschrieben ändert sich durch den Wirkstoff nicht.

Sofern sich die Wirkstoffkonzentration über die Rohrlänge ändern soll, kann ebenfalls mit Fäden unterschiedlicher Wirkstoffkonzentration gearbeitet werden.

## Patentansprüche

1. Polymerrohr hergestellt aus mindestens einem Polymerfaden, wobei der Polymerfaden einen Durchmesser von ≤ 50 µm aufweist.

2. Polymerrohr gemäß Anspruch 1, wobei das Polymerrohr einen Durchmesser von 0,5 - 15 mm aufweist.

3. Polymerrohr gemäß Anspruch 1 oder 2, wobei das Polymerrohr hergestellt ist aus mindestens einem bioresorbierbaren Polymer ausgewählt aus der Gruppe bestehend aus:
Poly(ε-caprolacton), Polyurethan, Polyhydroxybutyrat, Polylactonsäure, Polyglycolsäure, Polylactid, Polyglycolid, Copolymere der Polylactide und Polyglycolide.

4. Polymerrohr gemäß einem der Ansprüche 1 - 3, hergestellt aus einem Polymerfaden bestehend aus unterschiedlichen Polymeren oder hergestellt aus mindestens zwei Polymerfäden bestehend aus jeweils unterschiedlichen Polymeren.

5. Polymerrohr gemäß einem der vorherigen Ansprüche, wobei das Polymerrohr mindestens einen Wirkstoff umfasst und wobei das Polymerrohr über die Länge oder über die Wandstärke unterschiedliche Wirkstoffe und/oder Wirkstoffkonzentrationen aufweisen kann.

6. Verfahren zur Herstellung eines Polymerrohrs umfassend mindestens die folgenden Schritte:
a) Aufwickeln mindestens eines Polymerfadens mit einem Durchmesser von ≤ 50 µm; und
b) Verschmelzen des mindestens einen aufgewickelten Polymerfadens.

7. Verfahren zur Herstellung eines Polymerrohrs gemäß Anspruch 6, wobei der mindestens eine Polymerfaden durch Elektrospinnen oder Schmelzspinnen hergestellt wird.

8. Verfahren zur Herstellung eines Polymerrohrs gemäß Anspruch 6 oder 7, wobei der mindestens eine Polymerfaden in Schritt a) auf einem sich drehenden Dorn aufgewickelt wird.

9. Verfahren zur Herstellung eines Polymerrohrs gemäß Anspruch 8, wobei der mindestens eine Polymerfaden in Schritt a) beim Aufwickeln auf den sich drehenden Dorn gereckt und damit verfestigt wird.

10. Verfahren zur Herstellung eines Polymerrohrs gemäß einem der Ansprüche 6 - 8, wobei das Polymerrohr vor Schritt b) oder während des Verschmelzens gemäß Schritt b) oder nach Schritt b) auf einen Nenndurchmesser aufgedehnt und dabei verfestigt wird.

11. Verfahren zur Herstellung eines Polymerrohrs gemäß einem der Ansprüche 6 - 10, wobei der mindestens eine Polymerfaden in Schritt a) zielgerichtet lagenweise und/oder kreuzweise unter einem definierten Winkel und/oder gemäß einem vorgegebenen Muster aufgewickelt wird.

12. Polymerrohr erhältlich durch ein Verfahren gemäß einem der Ansprüche 6 - 11.

13. Stent hergestellt aus einem Polymerrohr gemäß einem der Ansprüche 1 - 5 oder 12.

14. Stent gemäß Anspruch 13, wobei der Innendurchmesser des Polymerrohrs dem Innendurchmesser des aufgedehnten Stents entspricht.

15. Stent, dessen Stentstreben aus verschmolzenen Teilstücken aus mindestens einem Polymerfaden bestehen, wobei der Polymerfaden einen Durchmesser von ≤ 50 µm aufweist.
